# EUROPÄISCHE PATENTSCHRIFT

(11) **EP 1 075 839 B1**
(45) Veröffentlichungstag und Bekanntmachung des Hinweises auf die Patenterteilung: **23.05.2012**
(21) Anmeldenummer: 00890210.8
(22) Anmeldetag: 04.07.2000
(51) Int. Cl.: A61K 31/718, A61K 47/36, A61K 9/00, A61P 7/08

(54) **Plasmaexpander, Blutverdünnungsmittel und Kryoprotektor, hergestellt unter Verwendung von Amylopektin-reicher Kartoffelstärke**
Plasma expander, blood diluent and cryoprotector produced using amylopectin-rich potato starch
Agent diluant du plasma, diluant sanguin et cryoprotecteur préparé en utilisant de l' amylopectine è partir d' amidon de pommes de terre

(30) Priorität: 10.08.1999 AT 137499
(43) Veröffentlichungstag der Anmeldung: 14.02.2001
(73) Patentinhaber: Coöperatie Avebe U.A., 9641 GK Veendam (NL)
(72) Erfinder: Grüll, Dietmar, Dr., 3442 Langenschönbichl (AT); Stifter, Ulrich, Dr, 3400 Klosterneuburg (AT)
(74) Vertreter: Hatzmann, Martin

(56) Entgegenhaltungen:
- EP-A- 0 402 724
- DE-A- 2 700 011
- DE-A- 3 313 600
- GB-A- 1 279 356
- US-A- 3 523 938
- G. S. NILSSON ET AL: "Determination of the Degree of Branching in Normal and Amylopectin Type Potato Starch with 1H-NMR Specroscopy" STARCH/STÄRKE, Bd. 48, Nr. 10, 1996, Seiten 352-357,

## Beschreibung

Die gegenständliche Erfindung betrifft die Verwendung von amylopektinreicher Stärke in der Medizin und Biologie zur Herstellung von Plasmaexpandern, Blutverdünnungsmitteln und Kryoprotektoren. Ebenso betrifft die Erfindung die Plasmaexpander, Blutverdünnungsmittel und Kryoprotektoren, die auf der Basis dieser Stärke hergestellt sind, sowie ein Verfahren zur Herstellung des bevorzugten Stärkederivats Hydroxyethylstärke für diesen Zweck.

Bereits im vorigen Jahrhundert wurde erkannt, daß Kochsalzlösung als Plasmaersatzmittel verwendet werden kann. Plasmaersatzmittel (Plasmaexpander) dienen zur Ergänzung des Blutes bei großem Blutverlust. Wegen starker Nebenwirkungen der Kochsalzlösung wurde nach besser verträglichen Substanzen gesucht. Nach der Jahrhundertwende diente Gelatinelösung als Plasmaersatz. Gummiarabikum-Lösung und Polyvinylalkohol konnten noch vor menschlichem Blut verwendet werden. Da menschliches Blut nur in begrenztem Ausmaß verfügbar, schlecht haltbar und nicht frei von Krankheitserregern ist, wurden weitere Chemikalien zur Verwendung als Plasmaersatzmitel getestet. Polyvinylpyrrolidon, Dextran und modifizierte Gelatine wurden eingesetzt, bevor der Versuch gemacht wurde, Plasmaexpander auf Stärkebasis zu erstellen. 1957 erschien die erste Publikation über Hydroxyethylstärke als Plasmaexpander.

Die EU-A1-0 402 724 beschreibt Hydroxyethylstärke auf der Basis amylopektinreicher Stärke als Plasmaexpander und beschreibt auch ein Verfahren zur Herstellung der HES. Dabei wird die HES durch Umsetzung der Stärke mit dem Hydroxyethylierungsmittel 2-Chlorethanol unter alkalischen Bedingungen gewonnen. Diese Patentanmeldung, die im Jahr 1989 eingereicht wurde, versteht unter amylopektinreicher Stärke in erster Linie Wachsmaisstärke. Es ist zwar auch Kartoffelstärke in diesem Zusammenhang angegeben, doch hat übliche Kartoffelstärke einen Amylosegehalt von etwa 20 bis 25 %. Eine amylopektinrelche Kartoffelstärke konnte damals nur durch Fraktionierung von üblicher Kartoffelstärke erhalten werden.
Nilsson et al. (Stärke 1996, 48(10), 352-357) beschreiben dass die Zahlenmittelwert der Kettenlänge von durch gentechnische verfahren gewonnen Amylopektin-Kartoffelstärke in der gleiche Bereich ist als Amylopektin fraktioniert von Kartoffelstärke.

Nichtsubstituierte Stärke kann als Plasmaexpander oder Blutverdünnungsmittel nicht verwendet werden, da die körpereigenen Amylasen zu rasch zu einem Abbau führen.

Künstliche Plasmaersatz- und Blutverdünnungsmittel unterscheiden sich von Blut, Plasma und kristalloiden Infusionslösungen grundsätzlich dadurch, daß sie kolloidale körperfremde Stoffe enthalten, die vom Organismus abgebaut und ausgeschieden werden müssen. Ein Plasmaersatzmittel muß die gestörte Hämodynamik nach einem akuten Blutverlust beseitigen, muß die körpereigenen Regulationsmechanismen unterstützen, im Kreislauf bis zu dessen Stabilisierung verweilen und anschließend unter minimaler Belastung des Stoffwechsels völlig eliminiert werden. Die heute verfügbaren körperfremden kolloidalen Plasmaersatzmittel sind heterodisperse Gemische hydrophiler Kolloide. Nach Infusion in das Gefäßsystem treten die Kolloide an die Stelle der Plasmaproteine und übernehmen deren Funktion im intravasalen Wasser- und Elektrolyttransport. Sie bewirken demnach eine Hämodilution mit entsprechender Hypoproteinämie, die solange anhält, bis der größte Teil der kolloidosmotisch wirksamen Partikel in den Extravasalraum abgeströmt bzw. durch die Nieren ausgeschieden ist.

Blutverdünnungsmittel dienen zur Behandlung peripherer und zerebraler Durchblutungsstörungen, indem sie die hämorheologischen Parameter günstig beeinflussen. Auch hier eignen sich körperfremde kolloidale Stoffe, wie Dextran und Stärkederivate. Eine solche Behandlung zur Hämodilution ist insbesondere bei arteriellen Verschlußkrankheiten und dergl. angezeigt.

Es wurde die folgende Reihe von Forderungen für eine wirksame und gefahrlose Anwendung von körperfremden kolloidalen Substanzen erarbeitet:
- Das Kolloid soll so beschaffen sein, daß es einen ausreichenden kolloidosmotischen Effekt bewirkt. Der kolloidosmotische Druck der Substanz im Lösungsmittel soll etwa von gleicher Größenordnung sein wie der des Plasmas.
- Die Molekülgröße des Kolloids soll dabei zugleich so bemessen sein, daß entweder das Gesamtkolloid oder seine Spaltprodukte im Glomerus gefiltert werden können, denn das Kolloid muß stets vollständig ausgeschieden oder abgebaut werden und darf nicht im Organismus gespeichert bleiben.
- Andererseits soll das Molekül groß genug sein, um eine ausreichende intravasale Verweildauer zu garantieren.
- Die Viskosität der Lösung soll der des Plasmas entsprechen.
- Das Kolloid soll keine toxischen, allergischen, anaphylaktoiden, antigenen und pyrogenen Reaktionen auslösen.
- Das Kolloid muß unverändert sterilisierbar sein.
- Neben diesen physiologischen Voraussetzungen müssen auch produktionstechnische Gesichtspunkte berücksichtigt werden. Das Plamaersatzmittel soll möglichst billig und mit konstanter, genau bekannter Eigenschaft technisch einfach und in ausreichender Menge herstellbar sein. Es soll lange haltbar und auch bei Temperaturschwankungen stabil sein, sodaß große Vorräte über lange Zeit eingelagert werden können. Die Lösung soll so beschaffen sein, daß sie jederzeit sofort einsatzbereit ist.

Die physikalisch-chemischen Eigenschaften der in den Plasmaersatzmitteln enthaltenen Kolloide sind für die Mehrzahl ihrer Wirkungen verantwortlich. Das Molekulargewicht ist das wesentliche Identifikationsmerkmal eines künstlichen Kolloids. Ein solches stellt im Gegensatz zu dem aus einheitlichen Molekülen aufgebauten Humanalbumin ein Gemisch verschieden großer Moleküle dar, sodaß infolge dieser Polydispersität die mitgeteilten Molekulargewichte der klinischen Kolloide stets als Mittelwerte zu betrachten sind.

In der Biologie ist ein anderes sehr wichtiges Einsatzgebiet von Stärkederivaten, insbesondere der Hydroxyethylstärke, deren Verwendung als Kryoprotektoren (Gefrierschutzadditiv) für lebende Zellen und Mikroorganismen. Beim Frieren und Tauen physiologischer Lösungen tritt eine starke Osmolaritätserhöhung auf, die die in der Lösung befindlichen Zellen zunächst dehydratisiert und bei genügend langer Einwirkungszeit lysiert. Kryoprotektoren beeinflussen diesen Prozeß durch Verminderung der osmotischen Aktivität der Lösung, Verlangsamung der Dehydratisierung und eine wirksame Stützung der Membran durch Anlagerung von Molekülen. Durch die Anlagerung von Molekülen an die Membran verringert sich deren Wasserdurchlässigkeit. Weiters müssen Kryoprotektoren die Eisbildung vermeiden, da durch Eiskristalle eine mechanische Zellschädigung eintreten kann. Bekannte Kryoprotektoren sind Polyhydroxyverbindungen wie Glycerin und Glykole, Zucker, Dextran und Dimethylsulfoxid.

Die in der Biologie verwendeten Kryoprotektoren werden in intrazelluläre- und extrazelluläre Stoffe eingeteilt. Substanzen mit niedrigem Molekulargewicht (wie z.B. Glycerin) können in die Zellen eindringen und agieren als intrazelluläre Substanz, während hochmolekulare Stoffe wie HES als extrazelluläre Wirkstoffe schützen. Intrazelluläre Substanzen wie Glycerin müssen z.B. bei der Verwendung als Blutgefrierschutzmittel vor der Anwendung am Menschen (durch Dialyse) entfernt werden. HES kann mit Blut appliziert werden.

Die vorliegende Erfindung ist nun dadurch gekennzeichnet, daß zur Herstellung von Plasmaexpandern, Blutverdünnungsmitteln und Kryoprotektoren eine Amylopektin-Kartoffelstärke verwendet wird, die aus durch Züchtung oder gentechnische bzw. andere molekularbiologische Verfahren hinsichtlich der Amylosebildung inhibierten Kartoffeln gewonnen wurde. Eine solche als Amylopektin-Kartoffelstärke bezeichnete Stärke hat einen Amylopektingehalt von über 95 Gew.%, vorzugsweise von über 98 %. Plasmaexpander, Blutverdünnungsmittel und Kryoprotektoren auf der Basis einer solchen Stärke entsprechen den gestellten Anforderungen in ausgezeichneter Weise und ihre Herstellung aus Amylopektin-Kartoffelstärke erfordert deutlich weniger Verfahrensschritte, wodurch vorteilhafterweise Angriffe und Schädigungen der Ausgangsstärke vermieden werden.

Am günstigsten wird die Amylopektin-Kartoffelstärke aus durch anti-sense-Technik oder durch Cosuppression hinsichtlich der Amylosebildung inhibierten Kartoffeln gewonnen.

Diese beiden Verfahren sind allgemein bekannt und bedürfen keiner weiteren Erklärung.
Es bestehen jedoch auch andere molekularbiologische Möglichkeiten, die Amylosesynthese zu inhibieren, wie z.B. Verfahren, die auf der Mutation der Kartoffelpflanze basieren.
Bevorzugt wird als Basis für Plasmaexpander, Blutverdünnungsmittel und Kryoprotektoren die Hydroxyethylstärke verwendet. Bei substituierten Stärken wie der HES, werden auch die molare und die durchschnittliche Substitution angegeben. Die molare Substitution (MS) gibt die durchschnittliche Anzahl von Substituenten (bei HES Hydroxyethylgruppen) pro Glucoseeinheit im Molekül an. MS kann auch Werte > 1 annehmen, da Substitutionen am C₂-, C₃- und C₆-Atom der Glucoseeinheiten (und hier wiederum Mehrfachsubstitutionen) möglich sind.

Die durchschnittliche Substitution (DS) gibt das Verhältnis der Anzahl substituierter (in Falle von HES hydroxyethylierter) Glucoseeinheiten im Molekül zu deren Gesamtzahl an. DS kann maximal den Wert 1 annehmen (jede Glucoseeinheit des Moleküls trägt dann mindestens eine Substituentengruppe (bei HES eine Hydroxyethylgruppe).

Ausgangsmaterial zur Herstellung von HES ist Stärke. Die aus üblichem Getreide oder natürlichen Kartoffeln gewonnenen Stärken stellen ein Gemisch aus den beiden Stärkeformen Amylopektin und Amylose dar. Amylose und Amylopektin sind ihrerseits wieder keine einheitlichen Substanzen, sondern Gemische von Polymeren mit unterschiedlichen Molekulargewichten und unterschiedlichen Glucose-Bindungen. Amylose besteht im wesentlichen aus unverzweigten Polysacchariden, in denen die Glucose in alpha-1,4-Bindungen vorliegt. Amylopektin hingegen ist ein stark verzweigtes Glucosepolymer, bei dem die Glucoseeinheiten neben den alpha-1,4-Bindungen an den Verzweigungsstellen in 1,6-Bindungen enthalten sind. Es hat sich gezeigt, daß Amylopektin stabilere Lösungen als Amylose ergibt, da Amylose zu unerwünschter Retrogradation, d.h. einem Wiederzusammenschluß bereits voneinander getrennter Ketten, neigt.

Die üblichen natürlichen Stärken enthalten unabhängig von der Pflanzenart, aus der sie gewonnen wurden, 15 bis 30% Amylose. Nur Maissorten des sogenannten Waxy-Typs liefern eine Stärke, die fast ausschließlich aus Amylopektin besteht. In seltenen Fällen kann eine amylopektinreiche Stärke auch aus sogenanntem Wachsreis oder aus Wachsgerste gewonnen werden.

Amylose und Amylopektin können durch chemische bzw. physikalische Verfahren voneinander getrennt werden. Doch sind diese Verfahren zu aufwendig und daher zu kostenintesiv, um die entstehende Amylopektin-Stärke für technische Zwecke einzusetzen.

Die Ermittlung des Amylosegehaltes bzw. des Amylpektingehaltes einer Stärke erfolgt nach: J.H.M. Hovenkamp-Hermenlink, J.N. DeVries, F. Adamse, E. Jacobsen, W. Witholt und W.J. Feenstra, Rapid estimation of the amylose amylopectin ratio in small amounts of tuber and leave tissue of the potatoe, Potatoe Res. 31, (1988), 241-246.

Für die Herstellung von HES muß die Ausgangsstärke gelöst werden. Diese Auflösung erfolgt mit Natronlauge, Salzsäure oder mit dem Enzym alpha-Amylase. Dabei tritt auch eine Molekulargewichtsreduktion auf. HES wird im allgemeinen in folgende Klassen eingeteilt: hochmolekular (Molekulargewicht 450000 Dalton), mittelmolekular (MW 200000 Dalton) und niedermolekular (MW 40000 Dalton). Beim Lösevorgang wird das gewünschte Molekulargewicht eingestellt. Wie bereits oben erwähnt, weist Amylose die unerwünschte Reaktion der Retrogradation, d.h. das irreversible Ausfallen aus der Lösung, auf. Auch die in der EU-A1 0 402 724 erwähnte Kartoffelstärke hat noch immer einen relativ hohen Amylosegehalt. Daher wurde bisher für die in der Medizin und Biologie verwendete HES bevorzugt Wachsmaisstärke eingesetzt. Allerdings weist Maisstärke einen höheren Protein- und Lipidgehalt als Kartoffelstärke auf.

| Stärke | Protein | Lipid |
|---|---|---|
| | % i. TS | |
| | | |
| Mais | 0,2 - 0,4 | 0,5 - 0,9 |
| Kartoffel | 0,05 - 0,1 | 0-0,1 |

Bei Verwendung von Maisstärke müssen diese störenden Substanzen entfernt werden. Kartoffelstärke ist dagegen eine sehr reine Stärke. Weiters benötigt der Mais ein wärmeres Klima. Wachsmais hat hohe Reifezahlen. Der Anbau bleibt auf Gunstlagen mit ausreichender Abgrenzung von normalem Mais beschränkt. Geringe Hektarerträge verursachen eine weitere Verteuerung. Die Kartoffel kann auch in schlechten Lagen mit hohen Hektareträgen angebaut werden. Vorteilhaft bei der Kartoffelstärke ist der natürliche relativ hohe Phosphatestergehalt. Dieser Phosphatestergehalt bewirkt ein erwünschtes höheres hydrodynamisches Volumen der HES. Vereinfacht ist das hydrodynamische Volumen das Volumen der Teilchen inclusive eingeschlossenem Lösungsmittel oder echt gebundenem Solvatationswasser.

| Stärke | Phosphorgehalt % i. TS |
|---|---|
| | |
| Kartoffel | 0,08 |
| Wachsmais | 0,007 (Phospholipide) |

Bei Verwendung von Wachsmaisstärke beginnt der Herstellungsprozeß mit einem Waschvorgang der Stärke. Natronlauge reduziert den Protein- und Pigmentgehalt. Bei Verwendung von Kartoffelstärke und Amylopektin-Kartoffelstärke kann dieser Reinigungsschritt entfallen. Dann wird die Stärke mit Salzsäure, Natronlauge oder alpha-Amylase abgebaut. Nach dem Lösen in Lauge erfolgt die Hydroxyethylierung. Früher wurde gasförmiges Ethylenoxid bei Normaldruck eingeleitet, moderne Verfahren arbeiten mit flüssigem Ethylenoxid unter Stickstoff-Schutzgasatmosphäre unter Druck. Durch Extraktion werden die Nebenprodukte entfernt. Eine Behandlung mit Aktivkohle entfärbt das Produkt. Zu kleine Moleküle werden durch eine Ultrafiltration entfernt. Nach einer Sprühtrocknung liegt das fertige Produkt vor.

Beispiel zur Herstellung von HES aus Amylopektin-Kartoffelstärke:
1 kg Amylopektin-Kartoffelstärke wird in 3,3 Liter 2 M Salzsäure bei 50°C suspendiert. Die Reaktionszeit beträgt je nach gewünschter Molekulargewichtsreduktion 2 bis 4 Stunden. Der Slurry wird auf 15°C abgekühlt und mit Natriumhydrogencarbonat neutralisiert. Die Stärke wird chloridfrei gewaschen. 1 kg der trockenen, säurehydrolysierten Amylopektin- Kartoffelstärke wird mit 141 ml destilliertem Wasser und 897 ml Isopropanol in einem druckfesten, verschließbaren Gefäß suspendiert. 25,6 g Natriumhydroxid werden zugegeben. Die Suspension wird 1 Stunde bei Raumtemperatur gerührt, dann wird auf 2°C abgekühlt. Die Luft wird mit Stickstoff aus dem Gefäß verdrängt. 256,4 ml 2°C kaltes, flüssiges Ethylenoxid werden zugesetzt und das Gefäß wird verschlossen. Der Reaktionsansatz wird zunächst gekühlt und gerührt, nach 1 Stunde wird die Kühlung und nach 2 Stunden das Rühren beendet. Die Reaktion wird 24 Stunden bei 37°C fortgesetzt. Durch Zugabe von Eisessig wird die Reaktion unter Rühren gestoppt. Die Flüssigkeit wird abgesaugt und das Produkt wird mit 1,3 l Ispropanol gewaschen. Glykole und Natriumacetat werden durch dreimaliges Waschen mit je 5,1 l wäßrigem Aceton (95 Vol %) und wäßrigem Ethanol (75 Vol %, wieder dreimal mit je 5,1 l) entfernt. Das Produkt wird getrocknet.

## Patentansprüche

1. Verwendung von amylopektinreicher Stärke in der Medizin und Biologie zur Herstellung von Plasmaexpandern, Blutverdünnungsmitteln und Kryoprotektoren, **dadurch gekennzeichnet, daß** die Stärke eine Amylopektin-Kartoffelstärke ist, die aus durch Züchtung oder gentechnische bzw. andere molekularbiologische Verfahren hinsichtlich der Amylosebildung inhibierten Kartoffeln gewonnen wurde.

2. Plasmaexpander, Blutverdünnungmittel und Kryoprotektor auf Basis von amylopektinreicher Stärke, **dadurch gekennzeichnet, daß** die Stärke eine Amylopektin-Kartoffelstärke ist, die aus durch Züchtung oder gentechnische bzw. andere molekularbiologische Verfahren hinsichtlich der Amylosebildung inhibierten Kartoffeln gewonnen wurde.

3. Plasmaexpander, Blutverdünnungmittel und Kryoprotektor nach Anspruch 2, **dadurch gekennzeichnet, daß** die Amylopektin-Kartoffelstärke aus durch anti-sense-Technik hinsichtlich der Amylosebildung inhibierten Kartoffeln gewonnen wurde.

4. Plasmaexpander, Blutverdünnungmittel und Kryoprotektor nach Anspruch 2, **dadurch gekennzeichnet, daß** die Amylopektin-Kartoffelstärke aus durch Cosuppressionstechnik hinsichtlich der Amylosebildung inhibierten Kartoffeln gewonnen wurde.

5. Plasmaexpander, Blutverdünnungmittel und Kryoprotektor nach einem der Ansprüche 2 bis 4, **dadurch gekennzeichnet, daß** die Amylopektin-Kartoffelstärke einen Amylopektingehalt von mindestens 95 %, vorzugsweise von mindestens 98 %, hat.

6. Plasmaexpander, Blutverdünnungmittel und Kryoprotektor nach einem der Ansprüche 2 bis 5, **dadurch gekennzeichnet, daß** die Amylopektin-Kartoffelstärke in Form der Hydroxyethylstärke vorliegt.

7. Verfahren zur Herstellung einer Hydroxyethylstärke aus amylopektinreicher Stärke, welche Hydroxyethylstärke als Basis für Plasmaexpander, Blutverdünnungmittel und Kryoprotektoren geeignet ist, **dadurch gekennzeichnet, daß** als Stärkeausgangsmaterial eine Amylopektin-Kartoffelstärke eingesetzt wird, die aus durch Züchtung oder gentechnische bzw. andere molekularbiologische Verfahren hinsichtlich der Amylosebildung inhibierten Kartoffeln gewonnen wurde, und daß diese Stärke mit Ethylenoxid in an sich bekannter Weise umgesetzt wird..

8. Verfahren nach Anspruch 7, **dadurch gekennzeichnet, daß** eine Amylopektin-Kartoffelstärke eingesetzt wird, die aus durch anti-sense-Technik hinsichtlich der Amylosebildung inhibierten Kartoffeln gewonnen wurde.

9. Verfahren nach Anspruch 7, **dadurch gekennzeichnet, daß** eine Amylopektin-Kartoffelstärke eingesetzt wird, die aus durch Cosuppressionstechnik hinsichtlich der Amylosebildung inhibierten Kartoffeln gewonnen wurde.

10. Verfahren nach einem der Ansprüche 7 bis 9, **dadurch gekennzeichnet, daß** eine Amylopektin-Kartofelstärke mit einem Amylopektingehalt von mindestens 95 %, vorzugsweise von mindestens 98 %, eingesetzt wird.

11. Verfahren nach einem der Ansprüche 7 bis 10, **dadurch gekennzeichnet, daß** die Amylopektin-Kartoffelstärke chemisch und/oder physikalisch und/oder enzymatisch vorbehandelt wird.

12. Verfahren nach Anspruch 11, **dadurch gekennzeichnet, daß** die Amylopektin-Kartoffelstärke mit Säure, vorzugsweise mit Salzsäure, vorbehandelt wird.

13. Verfahren nach Anspruch 11, **dadurch gekennzeichnet, daß** die Amylopektin-Kartoffelstärke mit Lauge, vorzugsweise mit Natronlauge, vorbehandelt wird.

14. Verfahren nach Anspruch 11, **dadurch gekennzeichnet, daß** die Amylopektin-Kartoffelstärke mit alpha-Amylase vorbehandelt wird.

## Claims

1. Use of amylopectin-rich starch in medicine and biology for the preparation of plasma expanders, blood-thinners and cryoprotectants, **characterized in that** the starch is an amylopectin potato starch extracted from potatoes, the amylose formation of which is inhibited by culturing or genetical or other molecular biological methods, respectively.

2. Plasma expander, blood-thinner and cryoprotectant on the basis of amylopectin-rich starch, **characterized in that** the starch is an amylopectin potato starch, extracted from potatoes, the amylose formation of which is inhibited by culturing or genetical or other molecular biological methods, respectively.

3. Plasma expander, blood-thinner and cryoprotectant according to claim 2, **characterized in that** the amylopectin potato starch is extracted from potatoes, the amylose formation of which is inhibited by antisense technique.

4. Plasma expander, blood-thinner and cryoprotectant according to claim 2, **characterized in that** the amylopectin potato starch is extracted from potatoes, the amylose formation of which is inhibited by co-suppression technique.

5. Plasma expander, blood-thinner and cryoprotectant according to one of claims 2 to 4, **characterized in that** the amylopectin potato starch has an amylopectin content of at least 95%, preferably of at least 98%.

6. Plasma expander, blood-thinner and cryoprotectant according to one of claims 2 to 5, **characterized in that** the amylopectin potato starch is available in form of hydroxyethyl starch.

7. Method for the preparation of a hydroxyethyl starch from amylopectin-rich starch, wherein the hydroxyethyl starch is suited as a basis for plasma expanders, blood-thinners and cryoprotectants, **characterized in that** an amylopectin potato starch is used as starch base material, which was extracted by potatoes, the amylose formation of which is inhibited by culturing or genetical or other molecular biological methods, respectively, and **in that** said starch is transformed with ethylene oxide in a manner known per se.

8. Method according to claim 7, **characterized in that** an amylopectin potato starch which is used extracted from potatoes, the amylose formation of which is inhibited by antisense technique.

9. Method according to claim 7, **characterized in that** an amylopectin potato starch which is used extracted from potatoes, the amylose formation of which is inhibited by co-suppression technique.

10. Method according to any of claims 7 to 9, **characterized in that** an amylopectin potato starch having an amylopectin content of at least 95%, preferably of at least 98%, is used.

11. Method according to any of claims 7 to 10, **characterized in that** the amylopectin potato starch is pre-treated chemically and/or physically and/or enzymatically.

12. Method according to claim 11, **characterized in that** the amylopectin potato starch is pre-treated with acid, preferably hydrochloric acid.

13. Method according to claim 11, **characterized in that** the amylopectin potato starch is pre-treated with lye, preferably soda lye.

14. Method according to claim 11, **characterized in that** the amylopectin potato starch is pre-treated with alpha-amylase.

## Revendications

1. Utilisation d'amidon riche en amylopectine en médecine et en biologie, pour la production de succédanés du plasma, de diluants sanguins et de cryoprotecteurs, **caractérisée en ce que** l'amidon est une fécule de pomme de terre d'amylopectine, qui a été obtenue à partir de pommes de terre dans lesquelles la formation d'amylose a été inhibée par culture ou par un procédé du génie génétique ou autre procédé biomoléculaire.

2. Succédané du plasma, diluant sanguin et cryoprotecteur à base d'amidon riche en amylopectine, **caractérisé en ce que** l'amidon est une fécule de pomme de terre d'amylopectine, qui a été obtenue à partir de pommes de terre dans lesquelles la formation d'amylose a été inhibée par culture ou par un procédé du génie génétique ou autre procédé biomoléculaire.

3. Succédané du plasma, diluant sanguin et cryoprotecteur selon la revendication 2, **caractérisé en ce que** la fécule de pomme de terre d'amylopectine a été obtenue à partir de pommes de terre dans lesquelles la formation d'amylose a été inhibée par une technique anti-sens.

4. Succédané du plasma, diluant sanguin et cryoprotecteur selon la revendication 2, **caractérisé en ce que** la fécule de pomme de terre d'amylopectine a été obtenue à partir de pommes de terre dans lesquelles la formation d'amylose est inhibée par une technique de co-suppression.

5. Succédané du plasma, diluant sanguin et cryoprotecteur selon l'une des revendications 2 à 4, **caractérisé en ce que** la fécule de pomme de terre d'amylopectine a une teneur en amylopectine d'au moins 95 %, de préférence d'au moins 98 %.

6. Succédané du plasma, diluant sanguin et cryoprotecteur selon l'une des revendications 2 à 5, **caractérisé en ce que** la fécule de pomme de terre d'amylopectine se présente sous la forme d'amidon d'hydroxyéthyle.

7. Procédé de production d'un amidon d'hydroxy-éthyle à partir d'amidon riche en amylopectine, ledit amidon d'hydroxyéthyle étant approprié comme base pour le succédané du plasma, le diluant sanguin et le cryoprotecteur, **caractérisé en ce que** l'on utilise une fécule de pomme de terre d'amylopectine comme matériau amidon de départ qui a été obtenue à partir de pommes de terre dans lesquelles la formation d'amylose est inhibée par culture ou par un procédé du génie génétique ou autre procédé biomoléculaire, et **en ce que** l'amidon réagit avec de l'oxyde d'éthylène de façon en elle-même connue.

8. Procédé selon la revendication 7, **caractérisé en ce que** l'on utilise une fécule de pomme de terre d'amylopectine qui a été obtenue à partir de pommes de terre dans lesquelles la formation d'amylose a été inhibée par une technique anti-sens.

9. Procédé selon la revendication 7, **caractérisé en ce que** l'on utilise une fécule de pomme de terre d'amylopectine qui a été obtenue à partir de pommes de terre dans lesquelles la formation d'amylose a été inhibée par une technique de co-suppression.

10. Procédé selon l'une des revendications 7 à 9, **caractérisé en ce que** l'on utilise une fécule de pomme de terre d'amylopectine qui a une teneur en amylopectine d'au moins 95 %, de préférence d'au moins 98 %.

11. Procédé selon l'une des revendications 7 à 10, **caractérisé en ce que** la fécule de pomme de terre d'amylopectine est prétraitée chimiquement et/ou physiquement et/ou enzymatiquement.

12. Procédé selon la revendication 11, **caractérisé en ce que en ce que** la fécule de pomme de terre d'amylopectine est prétraitée avec de l'acide, de préférence avec de l'acide chlorhydrique.

13. Procédé selon la revendication 11, **caractérisé en ce que en ce que** la fécule de pomme de terre d'amylopectine est prétraitée avec de la soude, de préférence avec de la soude caustique.

14. Procédé selon la revendication 11, **caractérisé en ce que en ce que** la fécule de pomme de terre d'amylopectine est prétraitée avec de l'alpha-amylase.
